# EUROPÄISCHE PATENTSCHRIFT

(11) **EP 2 948 421 B1**
(45) Veröffentlichungstag und Bekanntmachung des Hinweises auf die Patenterteilung: **13.03.2019**
(21) Anmeldenummer: 14701122.5
(22) Anmeldetag: 22.01.2014
(51) Int. Cl.: C07C 29/151, C25B 3/04, C07C 29/132, C07C 213/06

(54) **VERFAHREN ZUR IN-SITU UMSETZUNG VON CHEMISCH FIXIERTEM KOHLENDIOXID IN NIEDERMOLEKULARE KOHLENWASSERSTOFFE**
METHOD FOR IN-SITU CONVERSION OF CHEMICALLY FIXED CARBON DIOXIDE INTO LOW-MOLECULAR-WEIGHT HYDROCARBONS
PROCÉDÉ DE RÉACTION IN-SITU DE DIOXYDE DE CARBONE FIXÉ CHIMIQUEMENT POUR DONNER DES HYDROCARBURES À BAS POIDS MOLÉCULAIRE

(30) Priorität: 25.01.2013 DE 102013201246
(43) Veröffentlichungstag der Anmeldung: 02.12.2015
(73) Patentinhaber: Technische Universität Bergakademie Freiberg, 09599 Freiberg (DE)
(72) Erfinder: MERTENS, Florian, 09599 Freiberg (DE); RELLER, Christian, 32425 Minden (DE)
(74) Vertreter: Kailuweit & Uhlemann Patentanwälte Partnerschaft mbB
(86) Internationale Anmeldenummer: PCT/EP2014/051271
(87) Internationale Veröffentlichungsnummer: WO 2014/114686

(56) Entgegenhaltungen:
- WO-A1-2008/116799
- WO-A1-2012/015905
- OMAE ET AL: "Aspects of carbon dioxide utilization", CATALYSIS TODAY, ELSEVIER, NL, Bd. 115, Nr. 1-4, 30. Juni 2006 (2006-06-30), Seiten 33-52, XP027975905, ISSN: 0920-5861 [gefunden am 2006-06-30]
- RAMACHANDRAN ET AL.: "Kinetics of the Absorption of CO2 into Mixed Aqueous Loaded Solutions of Monoethanolamine and Methyldiethanolamine", IND. ENG. CHEM. RES., Bd. 45, 20. Januar 2006 (2006-01-20), Seiten 2608-2616, XP002723412, in der Anmeldung erwähnt
- RINKER ET AL.: "Absorption of Carbon Dioxide into Aqueous Blends of Diethanolamine and Methyldiethanolamine", IND. ENG. CHEM. RES., Bd. 39, 27. September 2000 (2000-09-27), Seiten 4346-4356, XP002723413, in der Anmeldung erwähnt

## Beschreibung

Die Erfindung betrifft ein Verfahren zur *in-situ* Umsetzung von chemisch fixiertem Kohlendioxid in niedermolekulare Kohlenwasserstoffe durch heterogenkatalytische oder elektrochemische Hydrierung. Die Erfindung eignet sich insbesondere zur stofflichen Nutzung von Kohlendioxid zur Erzeugung von Kraftstoffen und chemischen Grundchemikalien und der dadurch möglichen Speicherung elektrischer Energie.

Die Abtrennung des anfallenden Kohlendioxids (CO₂) von Biogasanlagen, aus Biomasseverbrennung und aus mit fossilen Brennstoffen betriebenen Verbrennungsanlagen erfolgt häufig durch Amin- oder Alkoholamin-Wäschen, die seit längerer Zeit in industriellen Verfahren zur CO₂-Abtrennung aus Prozessgasen verwendet werden. Zukünftig sind diese auch zur Abtrennung von CO₂ aus Rauchgasen von Kohlekraftwerken vorgesehen.

Die Regenerierung der Waschlösung erfolgt meist durch Entspannung oder durch eine Temperaturerhöhung auf ca. 100°C bis 130°C, wobei das chemisch gebundene CO₂ in die Gasphase entweicht. Für die Regenerierung kann z.B. Niederdruckdampf aus dem WasserDampf-Kreislauf eines Kraftwerks verwendet werden. Die thermische Regenerierung erfordert für MEA (Monoethanolamin) ca. 4 MJ/kg abgetrenntem CO₂, wodurch zum einen der Wirkungsgrad der Prozesse, die mit der beschriebenen CO₂-Abtrennung verbunden sind, vermindert wird. Zum anderen stellt der CO₂-Freisetzungsprozess einen eigenen Prozessschritt dar, der verfahrenstechnischen Aufwand bedeutet.

Die Einsparung der Freisetzung des CO₂ bei der stofflichen Verwertung, d.h. chemischen Nutzung durch CO₂-Reduktion, ist dabei von großem Interesse. Insbesondere die Herstellung von Kohlenwasserstoffen wie Methanol, Methan, Formaldehyden, Format, Methylformat und DME aus CO₂ ohne thermische CO₂-Abtrennung aus der Waschreagenz ist dabei von besonderem Interesse.

US 2010/0063320 A1 offenbart ein Verfahren zur Herstellung von Ameisensäure, als minderreduzierte organische Spezies, durch die homogene, katalytische Hydrierung von CO₂ mit H₂ unter Verwendung eines Katalysators der ein Metall der Gruppen 8 bis 10 des Periodensystems aufweist, im Speziellen Ruthenium-basierte Katalysatoren und in Gegenwart eines Amins. Durch die katalytische Umsetzung bildet sich ein Ammoniumformiat, wobei das Amin als Protonenfänger fungiert. Zur Regenerierung des Katalysators und zur Freisetzung der Ameisensäure, als gewünschtes Reaktionsprodukt, muss das als Intermediat gebildete Ammoniumformiat in einem zusätzlichen Schritt durch thermische Behandlung bei einer Temperatur zwischen 150 bis 200°C gespalten werden.

US 2011/0114502 A1 offenbart ein Elektrolyseverfahren zur Reduktion von CO₂ in einem wässrigen Medium, wobei das wässrige Medium als Katalysator ein heterozyklisches Arylamin mit einer Konzentration im millimolaren Bereich aufweist. Das CO₂ wird hierbei sprudelnd in das wässrige Medium eingeleitet. Um die Leitfähigkeit zu erhöhen, kann dem wässrigen Medium ferner ein Elektrolyt in Form von löslichen Salzen der Alkali- oder Erdalkalimetalle oder Ammoniumverbindungen zugesetzt sein.

Bekannt ist die Umsetzung von CO₂ mit wässrigen Alkoholamin-Lösungen, wobei als Amin hauptsächlich Monoethanolamin (MEA) **[1],** Diethanolamin (DEA) oder N-Methyldiethanolamin (MDEA) **[2]** verwendet wird. In diesen wässrigen Systemen verläuft die CO₂-Absorption unter Bildung von Hydrogencarbonat (nach Rinker *et al.* **[3],** Hagewiche *et al.* **[4]** und Versteeg *et al.* **[5]**).

WO 2008/116799 offenbart ein Verfahren zur Herstellung von Ameisensäure durch katalytische Hydrierung von Kohlendioxid mit Wasserstoff an einem Katalysator, enthaltend ein Metall der Gruppen 8 bis 10 des Periodensystems. In dem zweistufigen Prozess wird zunächst in Gegenwart eines Amins das entsprechende Ammoniumformiat erzeugt und dieses im zweiten Schritt durch Erhitzen in Ameisensäure und Amin gespalten. Bei dem Die Ameisensäure wird anschließend abdestilliert.

Aktuelle Forschungsprojekte beschäftigen sich mit der Verwendung von nichtwässrigen Alkoholaminlösungen (EEA, DEEA) bzw. Mischungen zur CO₂-Abtrennung **[6].** Die chemische Bindung des CO₂ erfolgt in diesen Medien intermediär unter Bildung eines R₂NH⁺ROCOO⁻ (mit R=Alkyl) Zwitterions das sich nach Deprotonierung durch eine Base in das jeweilige Carbamat umwandelt. Tertiäre Alkoholamine wie z.B. N,N-Diethylethanolamin (DEEA), N,N-Dimethylethanolamin (DMEA), N,N-Diisopropylethanolamin (DIPEA) eignen sich für druckgesteuerte Absorbtionsverfahren, da sowohl eine physikalische als auch chemische Speicherung von CO₂ vorliegt. Zur Erhöhung des Dampfdrucks werden hier oft Mischungen aus zweier dieser Substanzen verwendet. Der Verzicht auf Wasser erniedrigt die Korrosivität des Systems gegenüber Metall und auch gegenüber dem Katalysator **[7].**

Gegenwärtig spielt CO₂ keine nennenswerte Rolle als Mittel zur Energiespeicherung und zur Erzeugung chemischer Grundstoffe. CO₂ kann über den Sabatier-Prozess in Kohlenwasserstoffe umgewandelt werden. Das hierzu verwendete CO₂ muss über eine Abtrennung aus anderen Prozessen, wie. z.B. der Verbrennung fossiler Kraftstoffe oder von Biomasse, gewonnen werden. Hierzu gibt es die Möglichkeit der Auskondensation von CO₂ oder der CO₂-Wäsche. Bei letzterem Prozess wird in der Regel gasförmiges CO₂ von Chemikalien gebunden. Als solche Reagenzien kommen Amine, Alkoholamine und Natronlauge in Frage.

Die Anzahl der Publikationen mit dem Thema CO₂-Hydrierung zeigte in den letzten 5 Jahren einen sich stark entwickelnden Trend. Die bekannten Verfahren gliedern sich zum einen in Flüssigphasen- (>250°C **[8]**) und Gasphasenhydrierungen (>270°C **[9]**). Die Verfahren in der flüssigen Phase gliedern sich weiterhin in homogenkatalysierte und heterogenkatalysierte Methoden. Als katalytisch aktive Übergangsmetallkatalysatoren bei den homogenen Hydrierungen von CO₂ zu Methanol konnten Ruthenium-Pincer-Komplexe identifiziert werden. In der Flüssigphase werden als heterogene Katalysatoren zur Darstellung von Methanol hauptsächlich Cu-Katalysatoren mit Zusätzen von Zn, Zr, Ce, Al, Si, V, Ti, Ga, B, Cr verwendet **[10].** In der Gasphase erwiesen sich die bereits aus der Methanolsynthese bekannten Cu/ZnO Katalysatoren **[11,12]** als eine mögliche Wahl. Durch den Einsatz von Strukturpromotoren wie Ga₂O₃ **[13],** ZrO₂ **[14]** konnte die Stabilität der Cu/ZnO Katalysatoren erhöht werden. Als ein für die katalytische Aktivität entscheidendes Kriterium wird von den verschiedenen Autoren die Dispersion der Kupferspezies an der Oberfläche erwähnt. In der Literatur sind weitere Methoden zur Methanolherstellung bekannt, in denen Methanol auf indirektem Weg aus CO oder CO₂ und MeOH erhalten wird. Folgende Reaktionswege wurden dazu beschrieben:

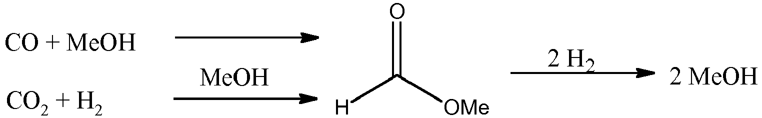

Ein entscheidender Nachteil aller bisher bekannten heterogen katalysierten Verfahren ist die geringe katalytische Aktivität und Produktivität der bekannten Katalysatoren bei Temperaturen T <270°C. Oftmals sind die eingesetzten Katalysatoren aufgrund der hohen Temperaturen nicht besonders selektiv gegenüber Methanol, so dass auch CO bei der Hydrierung gebildet wird **[15].**

Ein weiterer Nachteil der meisten dieser Methoden ist der hohe energetische Aufwand der Hydrierung im Verhältnis zur Ausbeute. Insbesondere Kupferkatalysatoren neigen einerseits zum thermischen Sintern bei Temperaturen >300°C; es finden Migrationseffekte an den aktiven Zentren der Katalysatoroberfläche statt, wodurch eine Deaktivierung des Materials eintritt **[16].** Die Verwendung von Promotoren zur Erhöhung der Stabilität der Struktur bzw. zur Erhöhung der Vergiftungsresistenz ist daher essentiell. Homogen katalysierte Verfahren besitzen den Nachteil der schlechten Abtrennbarkeit des Katalysators, so dass im Laufe des Prozesses mit hohen Verlusten zu rechnen ist.

Der Nachteil der gegenwärtigen Hydrierungsmethoden besteht darin, dass CO₂ zunächst als Reinstoff gewonnen werden muss. Die derzeit verfügbaren Methoden beinhalten wahlweise entweder die Auskondensation des CO₂ oder den Einsatz einer CO₂-Wäsche. Der erste Fall ist mit einem hohen Maß an einzusetzender Energie verbunden. Bei der CO₂-Wäsche wird das CO₂ durch thermische Behandlung wieder aus den Waschreagenzien ausgetrieben, was einen erhöhten energie- und verfahrenstechnischen Aufwand bedeutet. Ferner ist das reine CO₂ nur bei hohem Druck gasförmig oder flüssig bei Raumtemperatur mit vernünftigem Volumenbedarf lagerbar.

Bekannte Verfahren haben jedoch den erheblichen Nachteil, dass die Desorption des chemisch fixierten CO₂ aus den Waschreagenzien (d.h. Austreibung bzw. Freisetzung) unter einem erhöhten energie- und verfahrenstechnischen Aufwand und die Hydrierung des CO₂ in separaten Schritten ablaufen.

Aufgabe der Erfindung ist daher ein Verfahren zur *in-situ* Umsetzung von an organische, CO₂-bindende Komponenten enthaltende Waschreagenzien chemisch fixiertem Kohlendioxids in niedermolekulare Kohlenwasserstoffe, bei dem die beiden vorangegangenen Prozessschritte in einem Schritt ablaufen.

Erfindungsgemäß wird die Aufgabe gelöst durch ein Verfahren gemäß der Merkmale nach Anspruch 1.

Weitere vorteilhafte Ausgestaltungen enthalten die Merkmale der Unteransprüche.

lEine Ausgestaltung ist die *in-situ* Umwandlung von chemisch fixiertem Kohlendioxid in niedermolekulare Kohlenwasserstoffe, wobei das Kohlendioxid an ein organisches, CO₂-bindende Komponenten enthaltendes Waschreagenz chemisch fixiert und direkt katalytisch in Gegenwart eines heterogenen Metall-haltigen Katalysators oder elektrochemisch hydriert wird, und wobei im Anschluss die niedermolekularen Kohlenwasserstoffe von dem organischen, CO₂-bindende Komponenten enthaltenden Waschreagenz getrennt werden.

Als besonders vorteilhafte Ausgestaltung der vorliegenden Erfindung hat sich die *in-situ* Umwandlung von chemisch fixiertem Kohlendioxid (CO₂) in niedermolekulare Kohlenwasserstoffe herausgestellt, bei der
a) CO₂ in ein organisches, CO₂-bindende Komponenten enthaltendes Waschreagenz eingeleitet oder das organische, CO₂-bindende Komponenten enthaltende Waschreagenz mit CO₂ beaufschlagt wird, wobei das CO₂ an das organische, CO₂-bindende Komponenten enthaltende Waschreagenz chemisch fixiert und
b) direkt katalytisch oder elektrochemisch

in Gegenwart eines heterogenen Kupfer-basierenden Katalysators oder eines übergangsmetallhaltigen Katalysators, der Metalle der achten, neunten oder zehnten Gruppe des Periodensystems der Elemente aufweist, hydriert wird, und
wobei als Kathoden bei der elektrochemischen Hydrierung Kupfer-basierende oder übergangsmetallhaltige Elektrodenmaterialien eingesetzt werden, die Metalle der achten, neunten oder zehnten Gruppe des Periodensystems der Elemente aufweisen, und
wobei im Anschluss an die *in-situ* Umwandlung von chemisch fixiertem Kohlendioxid (CO₂) in niedermolekulare Kohlenwasserstoffe die niedermolekularen Kohlenwasserstoffe von dem organischen, CO₂-bindende Komponenten enthaltenden Waschreagenz getrennt werden.

Durch die Erfindung wird vorteilhaft das organische, CO₂-bindende Komponenten enthaltende Waschreagenz wieder regeneriert und liegt im Anschluss an die Hydrierung wieder in seiner ursprünglichen Struktur vor bzw. ist nicht irreversibel in seiner Funktion beeinträchtigt.

Vorteil des erfindungsgemäßen Verfahrens ist die Kombination der chemischen Fixierung des CO₂ mittels organischer, CO₂-bindende Komponenten enthaltender Waschreagenzien mit einer *in-situ* Hydrierung des chemisch gebundenen CO₂

Besonders vorteilhaft zeichnet sich das erfindungsgemäße Verfahren durch einen hohen CO₂-Umsatz bei einer gleichzeitig hohen Selektivität aus, sodass die Bildung unerwünschter Nebenprodukte, wie bspw. Kohlenmonoxid unterdrückt wird.

Das Verfahren ermöglicht eine Erweiterung der bisher eingesetzten CO₂-Absorption und eröffnet einen neuen Weg zu Kohlenwasserstoffen aus CO₂ insbesondere aber zu einer kostengünstigen Methanolsynthese und/oder anderer niedermolekularer Kohlenwasserstoffe. Durch dieses Verfahren lässt sich im Gegensatz zu den in der Literatur beschriebenen wasserhaltigen, Flüssigphasenhydrierungen eine bessere Ausnutzung des Reaktionsraumes ermöglichen, da die Waschreagenzien vielfach höhere Kapazitäten aufweisen und somit Kompressionsarbeit für CO₂ gespart werden kann. Aufgrund der hohen Siedepunktsunterschiede zwischen Kohlenwasserstoff und Waschreagenz ist eine leichte Abtrennbarkeit des Kohlenwasserstoffes möglich.

Neu ist die Einsparung der thermisch induzierten Freisetzung des CO₂ aus dem CO₂-beladenen Waschreagenz, da das an ein organisches, CO₂-bindende Komponenten enthaltendes Waschreagenz chemisch fixierte CO₂ erfindungsgemäß *in-situ* zu niedermolekularen Kohlenwasserstoffen hydriert wird. Neu ist zudem die energetische Kopplung von Hydrierung und Produktfreisetzung bzw. von Wasserstofferzeugung, Hydrierung und Produktfreisetzung, bzw. von CO₂-Einfang, Wasserstofferzeugung, Hydrierung und Produktfreisetzung, sowie verbleibender Kombinationen dieser Elemente.

Als CO₂-bindende Waschreagenzien werden bevorzugt Elektronendonatoren eingesetzt, die mit physikalisch gelöstem CO₂ eine chemische Bindung eingehen können.

Der Fachmann versteht unter dem Begriff "Hydrierung" definitionsgemäß jede Anlagerungsreaktion, bei der Wasserstoff an andere chemische Elemente oder Verbindungen addiert wird. Im Sinne der Erfindung ist demnach unter "Umwandlung von chemisch fixiertem Kohlendioxid in niedermolekulare Kohlenwasserstoffe" die Addition von Wasserstoff an Kohlenstoff bzw. Sauerstoff des chemisch fixierten Kohlendioxids zu verstehen. Der benötigte Wasserstoff kann beispielsweise basierend auf regenerativen Energiequellen durch Elektrolyse bereitgestellt werden.

Unter niedermolekularen Kohlenwasserstoffen werden im Sinne der vorliegenden Erfindung organische Verbindungen verstanden, die 1 oder 2 Kohlenstoffatome und Wasserstoffatome enthalten, wobei einzelne Wasserstoffatome durch Hydroxyl-Gruppen oder Carbonyl-Gruppen substituiert sein können. Niedermolekulare Kohlenwasserstoffe sind bspw. Methan, Methanol, Formaldehyd, Ethan oder Ethanol. Besonders bevorzugt ist der niedermolekulare Kohlenwasserstoff Methanol, Formaldehyd oder Methan, ganz besonders bevorzugt Methanol.

Überraschend hat sich gezeigt, dass durch das erfindungsgemäße Verfahren zur *in-situ* Umwandlung von chemisch fixiertem Kohlendioxid ausgewählte niedermolekulare Kohlenwasserstoffe mit einer hohen Selektivität erhalten werden. Vorzugsweise entsteht durch die erfindungsgemäße Umwandlung von chemisch fixiertem Kohlendioxid Methanol als niedermolekularer Kohlenwasserstoff mit einer Selektivität von mindestens 50%, besonders bevorzugt von mindestens 75%, ganz besonders bevorzugt von mindestens 90%.

Vorteilhaft wurde zudem beobachtet, dass bei Reaktionstemperaturen unterhalb von 200°C die Bildung unerwünschter, minderreduzierter Nebenprodukte, wie bspw. Kohlenmonoxid (CO) oder Formiat, unterdrückt ist. Eine besondere Ausführungsform des vorliegenden Verfahrens ist daher dadurch gekennzeichnet, dass die Reaktionstemperatur im Bereich zwischen 0 und maximal 200°C, besonders bevorzugt zwischen 10 und 180°C, ganz besonders bevorzugt zwischen 10°C und 150°C liegt.

Erfindungsgemäß wird ein organisches, CO₂-bindende Komponenten enthaltendes Waschreagenz, umfassend ein Alkoholamin und/oder ein Amin und/oder ein Arylamin und/oder beliebige Mischungen aus diesen, eingesetzt.

Aus ökologischen Gesichtspunkten werden in einer Ausgestaltung des erfindungsgemäßen Verfahrens bevorzugt tertiäre Alkoholamine (z.B. N,N-Diethylethanolamin DEEA, N,N-Dimethylethanolamin DMEA, N,N-Diisopropylethanolamin DIPEA) und/oder sekundäre Alkoholamine und/oder primäre Alkoholamine (z.B. MEA) als organisches, CO₂-bindende Komponenten enthaltendes Waschreagenz verwendet.

Vorteilhafterweise können tertiäre Alkoholamine, insbesondere DEEA aus regenerativen Rohstoffen gewonnen werden. Zudem lässt sich vorteilhaft das Reaktionsprodukt leicht von dem Alkoholamin, insbesondere hochsiedenden, tertiären Alkoholaminen (Siedepunkt > 100°C und oberhalb der Siedetemperatur von Methanol) nach der *in-situ* Hydrierung und/oder gegebenenfalls bereits während der *in-situ* Hydrierung unter den gegebenen Reaktionsbedingungen (insbesondere der Reaktionstemperatur) abtrennen.

In einer alternativ bevorzugten Ausgestaltung des erfindungsgemäßen Verfahrens ist das Alkoholamin ein Gemisch aus zwei separaten Verbindungen, wobei jede der Verbindungen nur eine der charakteristischen funktionellen Gruppen (d.h. Amin oder Alkohol) trägt. Vorteilhaft sind durch das Vorliegen zweier separater Verbindungen die charakteristischen funktionellen Gruppen voneinander entkoppelt.

Das Mischungsverhältnis zwischen Amin und Alkohol (in Gleichgewichtsteilen) in dem Alkoholamin liegt vorzugsweise in einem Bereich von 2:1 und 1:2. Besonders bevorzugt ist ein Mischungsverhältnis zwischen Amin und Alkohol von 1:1. Vorteilhaft hat sich hierbei gezeigt, dass bei dem Einsatz von tertiären Aminen unerwünschte Nebenreaktion (bspw. Bildung von Formiat) unterbunden wird.

In einer bevorzugten Ausgestaltung der vorliegenden Erfindung ist das Amin daher ein tertiäres Amin, ganz besonders bevorzugt ein nicht-heterozyklisches tertiäres Amin, wie bspw. Trimethylamin (TMA), Triethylamin (TEA), N,N-Diisopropylethylamin (DIEA), Tripropylamin (TPA), Tributylamin oder ein Gemisch dieser.

In einer alternativ bevorzugten Ausgestaltung ist das tertiäre Amin ein heterozyklisches tertiäres Amin, ganz besonders bevorzugt ein anelliertes heterozyklisches Amin, wie bspw. Diazabicycloundecen (DBU) oder 1,5-Diazabicyclo[4.3.0]non-5-ene (DBN) aber auch aromatische, tertiäre Amine, wie bspw. Pyridin, 2,6-Lutidin oder 4-Dimethylaminopyridin (DMAP) oder ein Gemisch dieser.

Bevorzugt ist der Alkohol ein kurzkettiger einwertiger Alkohol mit 1 bis 5 Kohlenstoffatomen (bspw. Methanol, Ethanol, Propanol oder Butanol) und/oder kurzkettiges Diol mit 2 bis 5 Kohlenstoffatomen (bspw. Ethylenglykol, 1,2-Propandiol).

Um die CO₂-bindenden Waschreagenzien mit CO₂ zu beladen, wird nach einer bevorzugten Ausgestaltung das CO₂ in das organische, CO₂-bindende Komponenten enthaltende Waschreagenz eingeleitet oder das organische, CO₂-bindende Komponenten enthaltende Waschreagenz mit CO₂ beaufschlagt. Dabei wird zunächst das eingeleitete CO₂ physikalisch gelöst und dann durch chemische Absorption wie z.B. durch eine nukleophile Addition unter Bildung eines Carbamates oder Zwitterions chemisch an das CO₂-bindende Waschreagenz fixiert.

Überraschend hat sich gezeigt, dass durch das erfindungsgemäße Verfahren zur Umwandlung von chemisch fixiertem Kohlendioxid in niedermolekulare Kohlenwasserstoffe, insbesondere durch die erfindungsgemäße chemische Fixierung des CO₂ am organischen, CO₂-bindende Komponenten enthaltenden Waschreagenz unter Bildung eines Carbamates oder Zwitterions, die Desorption des chemisch fixierten CO₂ aus den Waschreagenzien und die Hydrierung des CO₂ im Sinne eines Ersatzprozesses erfindungsgemäß in einem Prozessschritt ablaufen. Besonders vorteilhaft erlaubt somit das erfindungsgemäße Verfahren die Zusammenführung der beiden Reaktionsenthalpien, die sich gegenseitig kompensieren, sodass die zum erfindungsgemäßen Verfahren freiwerdende Reaktionswärme minimiert ist.

Besonders vorteilhaft wird somit durch die erfindungsgemäße chemische Fixierung des CO₂ am organischen, CO₂-bindende Komponenten enthaltenden Waschreagenz unter Bildung eines Carbamates oder Zwitterions die Hydriertemperatur für das an das CO₂-bindende Komponente bindende CO₂ erniedrigt.

Das erfindungsgemäße Verfahren hat somit den besonderen Vorteil, dass durch die nukleophile Addition von CO₂ unter Bildung eines Carbamates oder Zwitterions zum einen auftretende Reaktionswärmen minimiert sind und gleichzeitig auftretende Reaktionswärmen durch das organische, CO₂-bindende Komponenten enthaltende Waschreagenz abtransportiert werden, wodurch lokale Überhitzungen und eine thermisch bedingte Alterung des Katalysators (durch Migration) verhindert werden.

Die organischen, CO₂-bindende Komponenten enthaltenden Waschreagenzien werden bevorzugt substanziell wasserfrei eingesetzt. Substanziell wasserfreie organische, CO₂-bindende Komponenten enthaltende Waschreagenzien sind dadurch gekennzeichnet, dass sie geeigneter Weise zum Zeitpunkt vor Beginn des Verfahrens Wasser mit einem Gesamtanteil von maximal 10 Gew.-%, vorzugsweise maximal 5 Gew.-%, ganz besonders bevorzugt maximal 1 Gew.-% enthalten.

Nach einer bevorzugten Ausgestaltung des erfindungsgemäßen Verfahrens werden bevorzugt wasserarme organische, CO₂-bindende Komponenten enthaltende Waschreagenzien eingesetzt.

Wasserarme organische, CO₂-bindende Komponenten enthaltende Waschreagenzien sind Reagenzien, deren bevorzugte CO₂-bindende Wirkung durch die CO₂-bindende Komponenten bewerkstelligt wird. Bei der ablaufenden *in-situ* Hydrierungsreaktion wird Wasser als Produkt gebildet und wird somit zum Bestandteil des Systems. Wasser ist aber ausdrücklich kein Bestandteil des Wirkmechanismus. Die Teilchenanzahl des Wassers ist dabei stets kleiner als die Teilchenanzahl der organischen, CO₂-bindenden Komponente. Das Verhältnis zwischen der Teilchenanzahl des Wassers zu der Teilchenanzahl der organischen, CO₂-bindenden Komponente liegt dabei bevorzugt unterhalb eines Verhältnisses von 25:75 und besonders bevorzugt unterhalb 10:90.

Wasserarm ist das organische, CO₂-bindende Komponenten enthaltende Waschreagenz im Kontext dieser Erfindung dann, wenn das Verhältnis zwischen der Teilchenanzahl des Wassers zu der Teilchenanzahl der organischen, CO₂-bindenden Komponente dabei bevorzugt unterhalb eines Verhältnisses von 25:75, besonders bevorzugt unterhalb 10:90, ganz besonders bevorzugt unterhalb 5:95 liegt.

Nach einer weiterhin vorteilhaften Ausgestaltung des erfindungsgemäßen Verfahrens, erfolgt während des erfindungsgemäßen Verfahrens zur *in-situ* Umwandlung von chemisch fixiertem Kohlendioxid in niedermolekulare Kohlenwasserstoffe eine Abtrennung des als Produkt gebildeten Wassers.

Methoden zur Wasserabtrennung sind vielfach vorbeschrieben und dem Fachmann daher bestens bekannt. Vorteilhaft erfolgt die Abtrennung des als Produkt gebildeten Wassers destillativ bei erhöhter Temperatur (T>100°C) bzw. bei vermindertem Druck (p< 1 bar) aus dem organischen, CO₂-bindende Komponenten enthaltenden Waschreagenz. Gemäß einer besonders bevorzugten Ausgestaltung des erfindungsgemäßen Verfahrens erfolgt daher die direkt katalytische Hydrierung von chemisch fixiertem Kohlendioxid bei einer Reaktionstemperatur oberhalb von 100°C, ganz besonders bevorzugt im Bereich zwischen 100°C und 180°C.

Alternativ kann das als Produkt gebildete Wasser bspw. auch durch osmotische Membrantrennverfahren, mittels Adsorption (z.B. an Molekularsieben) oder Absorption von dem organischen, CO₂-bindende Komponenten enthaltenden Waschreagenz abgetrennt werden.

Ebenfalls vorteilhaft wird durch die Abtrennung des als Produkt gebildeten Wassers aus der Reaktionsmischung zudem das Gleichgewicht auf die Seite der Produkte (d.h. niedermolekulare Kohlenwasserstoffe) verschoben.

Mit dem beschriebenen Verfahren ist es in einer bevorzugten Ausgestaltung zudem möglich, CO₂ direkt in großtechnisch verwendeten Amin-Wäschen unter milden Reaktionsbedingungen von 10°C bis 150°C bei einem Wasserstoffdruck zwischen 1 bar bis 200 bar zu hydrieren. Das Verfahren ermöglicht somit die Herstellung von Methanol oder Formaldehyd und anderer Kohlenwasserstoffe (z.B. Methan) mittels einer heterogen katalysierten Hydrierung (in nicht wässrigen Alkoholaminen) oder einer elektrochemischen Reduktion des in chemischer Form gebundenen CO₂.

Bevorzugt beträgt der CO₂-Partialdruck in dem erfindungsgemäßen Verfahren zur *in situ* Hydrierung von chemisch gebundenen CO₂ 0,1 bis 200 bar, besonders bevorzugt 1 bis 100 bar. Vorteilhaft kann daher bspw. auch ein Gasgemisch aus Biogasanlagen eingesetzt werden, wobei das Gasgemisch lediglich einen Gesamtgehalt an CO₂ von mindestens 10 Vol.-%, vorzugsweise im Bereich von 15 bis 55 Vol.-% aufweist.

Bei der Hydrierung des chemisch fixierten Kohlendioxids durch den Einsatz von heterogenen Metallkatalysatoren werden nach einer bevorzugten Ausgestaltung der vorliegenden Erfindung bevorzugt Kupfer-basierende Katalysatoren eingesetzt, die keine Aminkomplexe bilden und signifikantes Katalysator-*leaching* zeigen.

Dabei wird in einer Weiterbildung des erfindungsgemäßen Verfahrens als Kupfer-basierender Katalysator bevorzugt ein Kupfer-Bor-Zirkonium-Wasserstoff Katalysator mit der allgemeinen Zusammensetzung CuₓB_{y}Zr_{z}Hₙ eingesetzt. Nach erfolgreich durchgeführten Zyklentests haben sich überraschenderweise als besonders geeignet Kupfer-basierende Katalysatoren mit der stöchiometrischen Zusammensetzung Cu₅₀₋₈₀B₅₋₁₀Zr₅₋₄₀H_{0,1-1} erwiesen.

Die strukturelle Integrität eines Katalysators (d.h. die Stabilität bzw. das Ausbleiben von Veränderung der Oberflächenstruktur bzw. chemischen Zusammensetzung des Katalysators) kann durch dem Fachmann bekannte physikalischer Charakterisierungsmethoden, wie bspw. Pulverdiffraktometrie, Auger-Spektroskopie, Röntgenfluoreszenzanalyse und/oder Röntgenphotoelektronenspektroskopie (XPS), nachgewiesen werden.

Für die heterogenkatalysierte Hydrierung lassen sich auch die aus der Methanolsynthese bekannten Cu/ZnO-Katalysatoren und andere geträgerte Cu-Katalysatoren verwenden, jedoch muss hier im Einzelfall sichergestellt werden, dass das Kupfer nicht durch die Bildung von Kupfer-Aminkomplexen in Lösung überführt wird. Andere gebräuchliche übergangsmetallische Hydrierkatalysatoren wie Ni, Pd, Pt usw. werden aufgrund ihrer geringen Selektivität, falls Methanol das gewünschte Produkt sein soll, nicht favorisiert. Als für die Reaktion ungeeignet erwiesen sich ebenfalls Cu° Nanopartikel, da diese zum einen zur starken Verklumpungserscheinungen neigten und zum anderen eine geringe katalytische Aktivität aufwiesen.

Nach einer weiteren Ausgestaltung der Erfindung werden als heterogenene metallhaltige Katalysatoren zur katalytischen Hydrierung hingegen bevorzugt übergangsmetallhaltige Hydrierungskatalysatoren eingesetzt, die Metalle der achten, neunten und/oder zehnten Gruppe des Periodensystems der Elemente (PSE) aufweisen, bei denen das Hydrierungsprodukt Methan sein soll. Metalle der achten, neunten und zehnten Gruppe des PSE sind dabei bevorzugt Ruthenium, Cobalt, Rhodium, Nickel und Platin. Die übergangsmetallhaltigen Hydrierungskatalysatoren zeigen kein signifikantes Katalysator-*leaching*.

Methan als Produkt hat den Vorteil, dass es sich in nichtrelevanten Mengen im organischen, CO₂-bindende Komponenten enthaltenden Waschreagenz löst und dadurch ohne weitere Reinigungsmethoden von dem organischen, CO₂-bindende Komponenten enthaltenden Waschreagenz abgetrennt werden kann.

Die chemische Fixierung des CO₂ am organischen, CO₂-bindende Komponenten enthaltenden Waschreagenz ist dadurch gekennzeichnet, dass ionische Spezies, inklusiv Zwitterionen bei der nukleophilen Addition des organischen, CO₂-bindende Komponenten enthaltenden Waschreagenz an das CO₂ auftreten. Dies erlaubt eine elektrochemische Hydrierung, wobei ebenfalls ein *in-situ* Verfahren bevorzugt bei einer Reaktionstemperatur T<150°C und einem Druck p<50 bar durchgeführt wird.

Bei der elektrochemischen Hydrierung von wasserfreien bzw. wasserarmen Alkoholamin-Lösungen mittels elektrokatalytischer Reaktionen werden nach einer bevorzugten Ausgestaltung der vorliegenden Erfindung mehrteilige Elektrolysezellen verwendet, bei denen die Elektrolysezellen durch eine protonenpermeable (H⁺) Membran unterteilt sind.

Der Kathodenraum enthält dazu das organische, CO₂-beladene Komponenten enthaltende Waschreagenz, in das CO₂ eingeleitet wird. Als Kathoden werden Kupfer-basierende oder übergangsmetallhaltige Elektrodenmaterialien eingesetzt, die Metalle der achten, neunten und/oder zehnten Gruppe des Periodensystems der Elemente aufweisen und kein signifikantes Leaching zeigen.

Im Anodenraum befindet sich Wasser, welches zu Sauerstoff und unter der Bildung von Hydroniumionen reagiert.

6*H*₂*O* → 4*H*₃*O*⁺ + *O*₂ + 4*e*⁻

Die an der Anode gebildeten Protonen diffundieren durch die Membran zur Kathode, wo sie zu Wasserstoff reduziert werden. Der entstehende naszierende Wasserstoff reduziert dann das im Amin absorbierte CO₂. Die Methode bietet den Vorteil, dass Strom aus regenerativen Energien (Windkraft) für die Elektrolyse und der Erzeugung von Wasserstoff verwendet wird.

Bei dieser Ausgestaltung des vorliegenden Verfahrens wird der Wasserstoff direkt während der *in situ* Hydrierung als atomarer Wasserstoff (sog. naszendierender Wasserstoff) über die Kathode bereitgestellt. Somit kann vorteilhaft auf die Einleitung oder Beaufschlagung von molekularem Wasserstoff (H₂) verzichtet werden.

Bevorzugt erfolgt die elektrochemische Hydrierung bei einem Gesamtdruck im Bereich zwischen 0,1 bis 50 bar.

Vorteilhaft hat sich ferner bei der elektrochemischen Hydrierung gezeigt, dass durch das negative Elektrodenpotential der Kathode während der erfindungsgemäßen *in situ* Hydrierung einer unerwünschten Bildung von Aminkomplexen und somit dem signifikanten Katalysator-*leaching* zusätzlich entgegengewirkt wird, sodass die strukturelle Integrität des Katalysators (d.h. Kathode) erhalten bleibt.

Wie oben bereits ausgeführt, kann die strukturelle Integrität des Katalysators (d.h., keine Veränderung der Oberflächenstruktur bzw. chemischen Zusammensetzung) durch dem Fachmann bekannte physikalische Charakterisierungsmethoden, wie bspw. Pulverdiffraktometrie, Auger-Spektroskopie, Röntgenfluoreszenzanalyse und/oder Röntgenphotoelektronenspektroskopie (XPS), nachgewiesen werden.

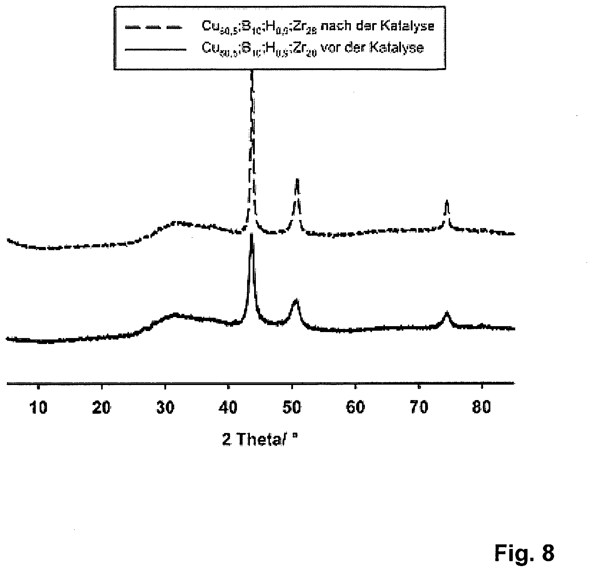

Ferner hat sich gezeigt, dass nach einer erfindungsgemäßen Ausgestaltung des Verfahrens bei dem das substanziell wasserfreie, organische, CO₂-bindende Komponenten enthaltende Waschreagenz ein Alkoholamin ist, auf den Zusatz eines Elektrolyten (Leitsalz) in Form von Salzen der Alkali- oder Erdalkalimetalle oder Ammoniumverbindungen verzichtet werden kann (siehe Fig. 8).

Das erfindungsgemäße Verfahren zur *in-situ* Umwandlung von chemisch fixiertem Kohlendioxid ermöglicht die Herstellung von Methanol oder Formaldehyd und/oder anderer niedermolekularer Kohlenwasserstoffe (z.B. Methan) durch eine heterogen katalysierte Hydrierung (in nicht wässrigen Alkoholaminen) oder eine elektrochemische Reduktion des in chemischer Form gebundenen CO₂.

Vorteilhaft entstehen bei der Hydrierung freie, ungebundene niedermolekulare Kohlenwasserstoffe und freie organische, CO₂-bindende Komponenten enthaltende Waschreagenzien, die nicht mit CO₂ beladen sind.

Die niedermolekularen Kohlenwasserstoffe müssen von dem organischen, CO₂-bindenden Komponente enthaltenden Waschreagenz gegebenenfalls durch ein Extraktionsverfahren, oder ein Destillationsverfahren oder mittels Chromatographie abgetrennt werden.

Anhand nachfolgender Ausführungsbeispiele und beigefügter Darstellungen wird die Erfindung näher erläutert. Dabei zeigen:
**Fig. 1****:** Gesamter Stoffmengenanteil des physikalisch und chemisch absorbierten CO₂ in der flüssigen Phase für Ausführungsbeispiel 1 (DEEA).
**Fig. 2****:** ¹³C-NMR (CDCl₃) nach 12 h der Hydrierung aus Ausführungsbeispiel 1 (unteres Spektrum) und reinem DEEA (oberes Spektrum).
**Fig. 3****:** Massenspektrum der Gasphase : Hauptbestandteile: Wasserstoff (2) und CO₂ (44) Nebenbestandteile: Methan (16), CO (28)
**Fig. 4****:** ¹³C-NMR (CDCl₂) der durch Vakuumdestillation abgetrennten leicht flüchtigen Bestandteile. Der kommerzielle Katalysator reagiert mit dem DEEA unter Bildung vieler Nebenprodukte.
**Fig. 5****:** Gaschromatogramm nach einer Reaktionszeit von 12 h; Hauptbestandteile: Methanol (MeOH) und DEEA.
**Fig. 6****:** Zwei übereinandergelegte Pulverdiffraktogramme des kupferbasierenden Katalysators Cu/ZnO-Al₂O₃ vor und unmittelbar nach der erfindungsgemäßen Umsetzung von chemisch fixiertem Kohlendioxid.
**Fig. 7****:** XPS-Messungen ausgesuchter Elektronenübergänge des kupferbasierenden Katalysators Cu/ZnO-Al₂O₃ vor und unmittelbar nach der erfindungsgemäßen Umsetzung von chemisch fixiertem Kohlendioxid.
**Fig. 8****:** Zwei übereinandergelegte Pulverdiffraktogramme des kupferbasierenden Katalysators Cu_{60,5};B₁₀;H_{0,9};Zr_{28,6} vor und unmittelbar nach der erfindungsgemäßen Umsetzung von chemisch fixiertem Kohlendioxid.
**Fig. 9****:** Leitfähigkeitsmessung in 150 ml nichtwässrigem DEEA bei Temperaturen von 30°C, 50°C und 100°C.

### Ausführungsbeispiel 1

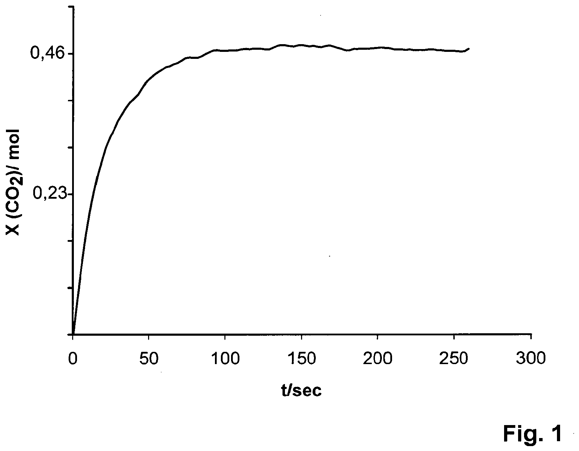

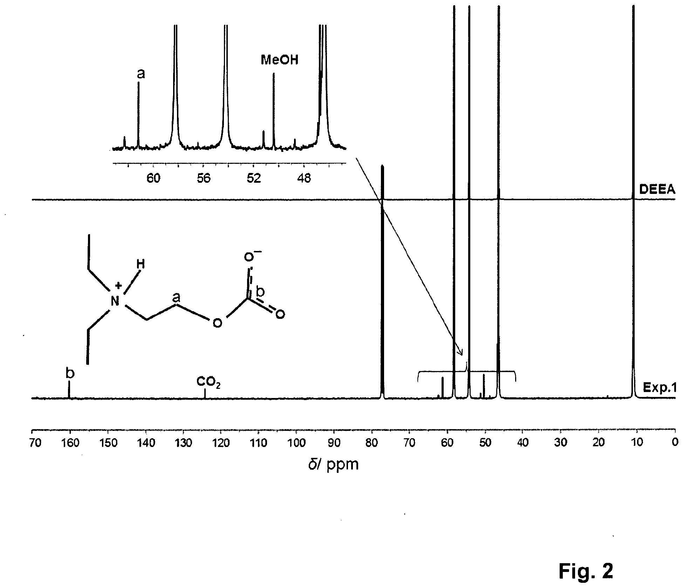

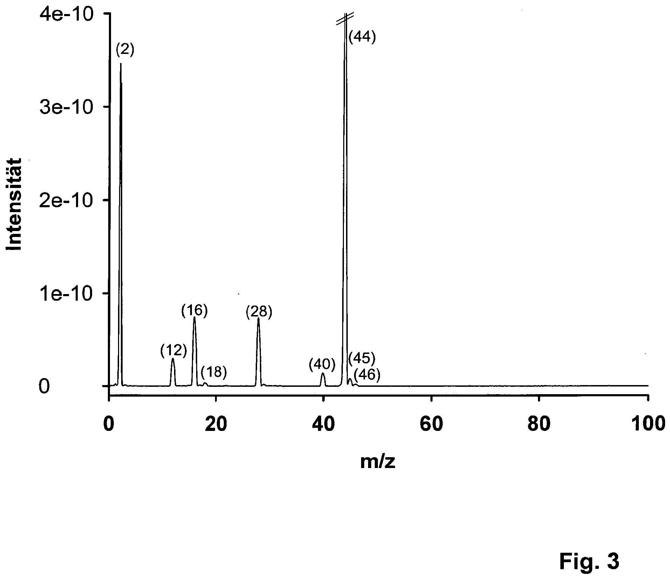

In einem 20 ml Hastalloy C22 Autoklaven wurden 10 ml DEEA und 100 mg des CuBHxZr Katalysators gemäß **Fig. 8****,** wenn nicht anders beschrieben, eingefüllt und die Mischung auf die gewünschte Reaktionstemperatur temperiert. Danach erfolgte die Zugabe von gasförmigem CO₂ bis zur vollständigen Sättigung des Amins unter CO₂ Überdruck (p_{CO2}) in der Gasphase **(****Fig. 1****).** Im zweiten Schritt erfolgte die Zugabe von H₂ bis zur vollständigen Sättigung und einem partialem Überdruck von H₂ (p_{H2}) in der Gasphase. Die Reaktion wird gestartet, nachdem Wasserstoff in die Apparatur gepresst und der Rührer in Betrieb gesetzt wurde. Die Hydrierung wird über eine Reaktionszeit (t) von 12 h, wenn nicht anders beschrieben, bei der Reaktionstemperatur T durchgeführt und danach der Umsatz an Methanol mittels ¹³C-NMR Spektroskopie **(****Fig. 2****)** bestimmt und die überstehende Gasphase mit einem Prozessmassenspektrometer **(****Fig. 3****)** analysiert.

Aufgrund der Empfindlichkeit des verwendeten Katalysators gegenüber Luftsauerstoff sind alle Reaktionen unter Inertgas und mit getrockneten Reagenzien bzw. Lösungsmitteln durchgeführt worden.

### Versuchsbedingungen:

Analog zu Ausführungsbeispiel 1 wurden folgende Versuchsbedingungen gewählt: T= 30°C, p_{CO2}=41bar, p_{H2} = 20bar, t=12h (10 ml DEEA)
X_{abs}(CO₂)=0,46 (Abb.1)
Es wurde ein Umsatz zu MeOH von 60% bezogen auf die in dem DEEA physikalisch und chemisch gelöstem CO₂ mit einer Selektivität von 95% erreicht. Als Nebenprodukte in der Gasphase wurden CO und CH₄ in Spuren detektiert **(siehe** **Fig. 2****).**

### Ausführungsbeispiel 2

### Versuchsbedingungen:

Analog zu Ausführungsbeispiel 1 wurden folgende Versuchsbedingungen gewählt: T=100°C, p_{0,CO2}=20bar, p_{0,H2} = 70bar, t=24h (20 ml DEEA) 4,1g R3-11/G Katalysator CuO/ZnO (3x5mm) (1,43 g Cu). Der Katalysator wurde zuvor 12h im Wasserstoffstrom bei 260°C aktiviert. X_{abs}(CO₂)=0,043.
Es wurde ein Umsatz zu MeOH von 3% bezogen auf die in dem DEEA physikalisch und chemisch gelöstem CO₂ mit einer Selektivität von 50% erreicht. Als Nebenprodukte wurden Methylformat in der flüssigen Phase und CO und CH₄ in der Gasphase detektiert.

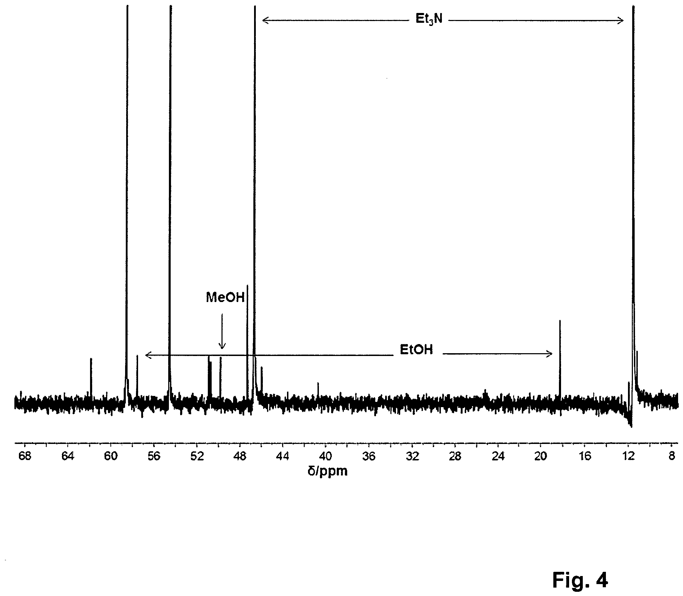

Das angeführte ¹³C-NMR Spektrum in **Fig. 4** der leicht flüchtigen Bestandteile des Reaktionsansatzes zeigt eine teilweise Zersetzung des DEEA unter Bildung von Et₃N und Ethanol. Es liegen neben Methanol ebenfalls viele Nebenprodukte vor.

### Ausführungsbeispiel 3

### Versuchsbedingungen:

Analog zu Ausführungsbeispiel 1 wurden folgende Versuchsbedingungen gewählt: T=50°C, p_{CO2}=50 bar, p_{H2} = 30 bar, t=30 h (20 ml DEEA) 200mg R3-11/G Kat (gepulvert und nicht aktiviert) Für dieses Experiment wurde ein Umsatz an MeOH von 5% mit einer Selektivität von 50% erreicht. Als Nebenprodukte in der Gasphase wurden CO und CH₄ detektiert.

### Ausführungsbeispiel 4 - Herstellung des Katalysators

6 g CuNO₂·3H₂O (0,0248 mol) und 1,59 g ZrCl₂O (0,0089mol) wurden in einem 500 ml Dreihalskolben in 250 ml dest. Wasser gelöst. Unter starkem Rühren und ständiger Argonspülung des Reaktionsgefäßes erfolgte dann die Zugabe eine Lösung aus 9,2 g (0,248 mol) NaBH₄ und 1g NaOH gelöst in 100 ml dest. Wasser über eine Spritzenpumpe (0,1 l/h). Nach der Zugabe wurde der schwarze Nd. mit einer Schlenkfritte abfiltriert und 3x mit 50 ml Wasser, 3x mit 20 ml EtOH (abs.) und zuletzt 3x mit 15 ml Et₂O gewaschen. Im Anschluss wurden letzte Lösungsmittelreste im Vakuum bei 1x10⁻³mbar entfernt.

### Katalysatorcharakterisierung:

Der frisch synthetisierte Katalysator hatte eine elementare Zusammensetzung von CuₓB_{y-}Zr_{z}Hₙ.

### Ausführungsbeispiel 4

### Versuchsbedingungen:

T= 170°C, p_{0,CO2}=20 bar, p_{0,H2} = 60 bar, t₁=6 h, t₂=12 h (20 ml DEEA), 300 mg Kupferbasierter Katalysator Cu/ZnO-Al₂O₃.

Nach einer Reaktionszeit t₁=6 h wurde ein Umsatz des in dem DEEA physikalisch und chemisch gelöstem CO₂ zu MeOH von 30% und nach t₂=12 h von 60% ermittelt. In beiden Fällen betrug die Selektivität der Umsetzung von CO₂ zu MeOH 90%.

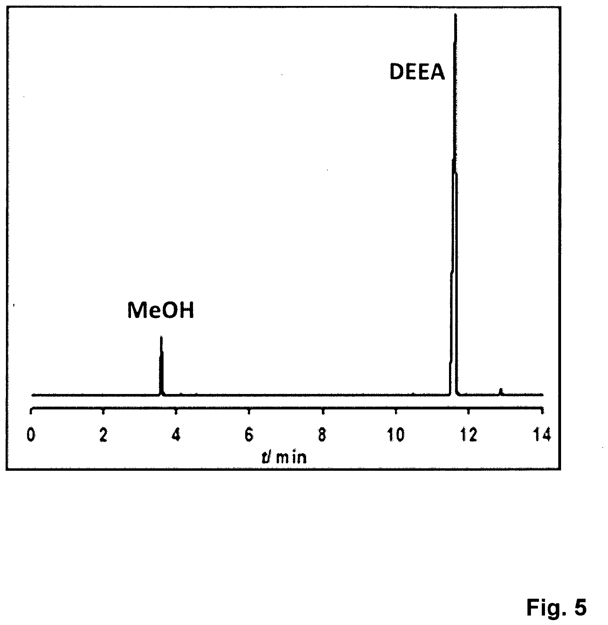

Nach der Hydrierung unter den Reaktionsbedingungen aus Beispiel 4 zeigt das entsprechende Gaschromatogramm (Fig. 5) lediglich zwei Signale, welche dem Methanol (MeOH) oder DEEA zugeordnet werden konnten.

### Ausführungsbeispiel 5 - Nachweis der Stabilität von Kupfer-basierten Katalysatoren

Um die Stabilität der Katalysatoren gegenüber Aminen zu ermitteln, wurden nachfolgend Kupfer-basierende Katalysatoren vor und nach der erfindungsgemäßen Umwandlung von chemisch fixiertem Kohlendioxid (Hydrierungsreaktion) mittels Pulverdiffraktometrie und Röntgenphotoelektronenspektroskopie (XPS-Messung) auf den Erhalt ihrer strukturellen Zusammensetzung untersucht.

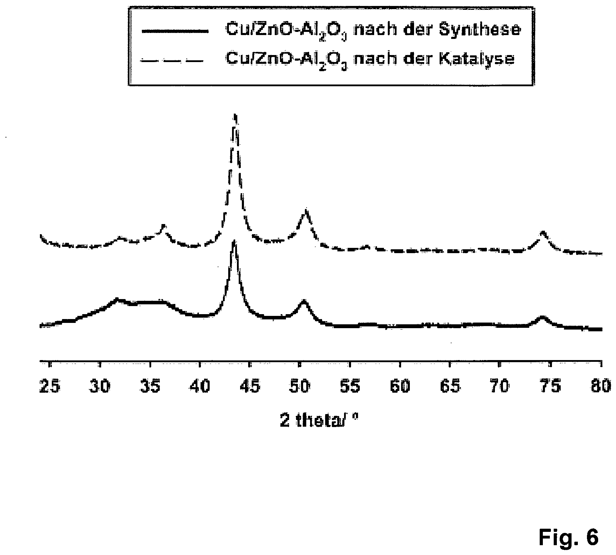

**Fig. 6** zeigt zwei übereinandergelegte Pulverdiffraktogramme des kupferbasierenden Katalysators Cu/ZnO-Al₂O₃. Das untere Diffraktogramm (durchgängiger Graph) zeigt die kristalline Struktur des Katalysators direkt nach dessen Synthese. Das darüber liegende Diffraktogramm (gestrichelter Graph) zeigt die Struktur des Katalysators nach der erfindungsgemäßen Hydrierungsreaktion von CO₂ zu Methanol in Gegenwart von 20 ml DEEA bei einer Reaktionstemperatur von 150°C. Aus Fig. 5 ist erkennbar, dass sich die Struktur des Katalysators (absoluter Gehalt von 300 mg) auch nach der Hydrierungsreaktion über einen Zeitraum von 24 h nicht verändert und keine weiteren Phasen bzw. Zersetzungsprodukte nachweisbar sind.

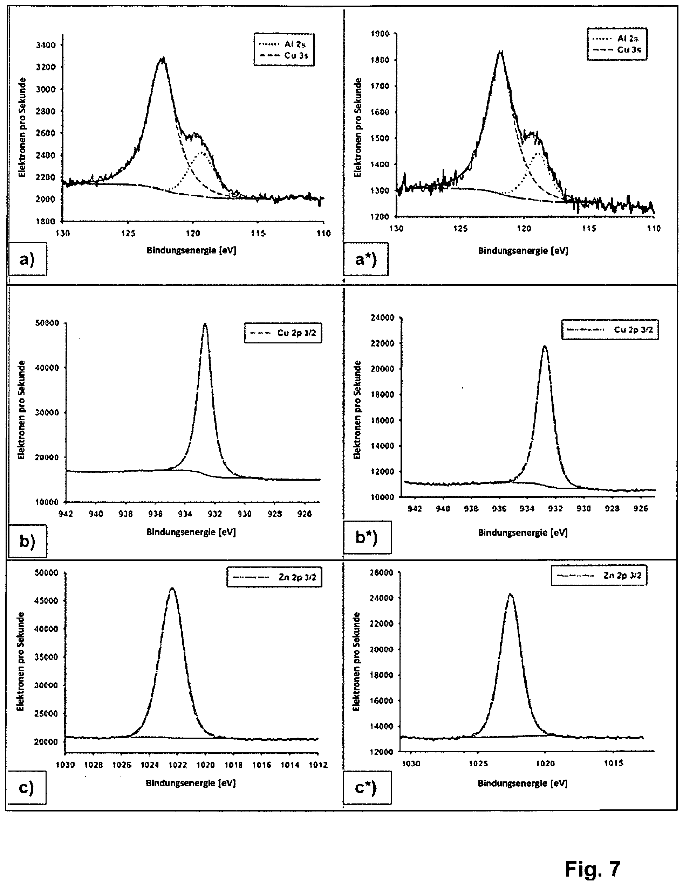

**Fig. 7** zeigt korrespondierend zu den vorgenannten Versuchsbedingungen für den kupferbasierenden Katalysator Cu/ZnO-Al₂O₃ die Datensätze von zwei XPS-Messungen, wobei die Datensätze der beiden XPS-Messungen jeweils Oberflächenzusammensetzung des Katalysators vor der erfindungsgemäßen Hydrierungsreaktion (linksseitig) bzw. nach der erfindungsgemäßen Hydrierungsreaktion (rechtsseitig) darstellen. Zur Analyse wurden die folgenden Elektronenübergänge bzw. deren Signale ausgewertet:
a) Al 2s, Cu 3s,
b) Cu 2p 3/2 und
c) Zn 2p 3/2.

Die Gegenüberstellung der Ergebnisse verdeutlicht, dass keine Veränderung der Oberflächenstruktur bzw. -zusammensetzung des Kupfer-basierenden Katalysators durch die erfindungsgemäße Hydrierungsreaktion von CO₂ zu Methanol in Gegenwart von DEEA bei 150°C identifizierbar ist. Der kupferbasierende Katalysator ist demnach stabil gegenüber dem Auswaschen durch DEEA.

**Fig. 8** zeigt zwei übereinandergelegte Pulverdiffraktogramme des kupferbasierenden Katalysators Cu_{60,5};B₁₀;H_{0,9};Zr_{28,6}. Hieraus ist ersichtlich, dass sich die Struktur des verwendeten Katalysators direkt nach dessen Synthese (durchgängiger Graph) und nach der erfindungsgemäßen Hydrierung von CO₂ zu Methanol in Gegenwart von 20 ml DEEA bei einer Reaktionstemperatur von 150°C (gestrichelter Graph) nicht unterscheiden. Der kupferbasierende Katalysator (absoluter Gehalt von 1,42 *µ*g) ist demnach mindestens über einen Zeitraum von 24 h unter den gegebenen Reaktionsbedingungen stabil gegenüber dem Auswaschen durch DEEA.

### Ausführungsbeispiel 6 - elektrochemische Hydrierung

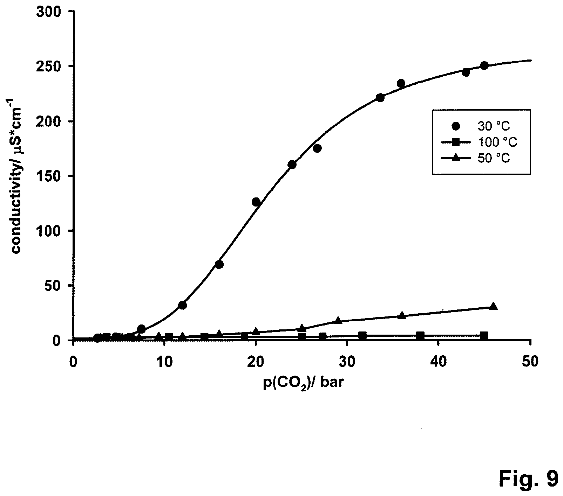

Zur Ermittlung der Leitfähigkeit (vgl. **Fig. 9**) einer mit CO₂ beaufschlagten organischen, CO₂-bindende Komponenten enthaltenden Waschreagenz, wurden 150 ml DEEA ohne weitere Zusätze, insbesondere ohne Elketrolyten, mit einem Partialdruck (p_{CO2}) von 45 bar beaufschlagt.

Während bei einer Temperatur von 100°C die Leitfähigkeit (in S*cm⁻¹) minimal ist, steigt diese bei einer Temperatur von 50°C auf 35 S*cm⁻¹ leicht an und beträgt bei einer Versuchstemperatur von 30°C über 250 S*cm⁻¹.

### Zitierte Literatur

**[1]** A. J. Kidnay, W. R. Parrish, Fundamentals of Natural Gas Processing 2006, CRC Press: Boca Ranton, FI.
**[2]** N. Ramachandran, A. Aboudheir, R. Idem, P. Tontiwachwuthikul, Industrial & Engineering Chemistry Research 2006, 45, 2608-2616.
**[3]** E. B. Rinker, S. S. Ashour, O. C. Sandall, Ind. Eng. Chem. Res 2000, 39, 4346-4356.
**[4]** D. P. Hagewiesche, S. S. Ashour, H. A. Al-Ghawas, O. C. Sandall, Chemical Engineering Science 1995, 50, 1071-1079.
**[5]** G. F. Versteeg, W. P. M. van Swaaij, Chemical Engineering Science 1988, 43, 573-585.
**[6]** P. D. Vaidya, E. Y. Kenig, Chemie Ingenieur Technik 2012, 84, 475-483.
**[7]** J. E. Rainbolt, P. K. Koech, C. R. Yonker, F. Zheng, D. Main, M. L. Weaver, J. C. Linehan, D. J. Heldebrant, Energy Environ. Sci 2011, 4, 480-484.
**[8]** S.-i. Fujita, S. Moribe, Y. Kanamori, M. Kakudate, N. Takezawa, Applied Catalysis A: General 2001, 207, 121-128.
**[9]** C.-H. Kim, J. Lee, D. L. Trimm, Topics in Catylysis 2003, 22, 319-324.
**[10]** W. Wang, S. Wang, X. Ma, J. Gong, Chem. Soc. Rev 2011, 40, 3703-3727.
**[11]** S.-i. Fujita, S. Moribe, Y. Kanamori, M. Kakudate, N. Takezawa, Applied Catalysis A: General 2001, 207, 121-128.
**[12]** J. Nakamura, Y. Choi, T. Fujitani, Topics in Catalysis 2003, 22, 277-285.
**[13]** J. Toyir, P. R. de La Piscina, J. L. G. Fierro, N. Homs, Applied Catalysis B: Environmental 2001, 29, 207-215.
**[14]** F. Arena, K. Barbera, G. Italiano, G. Bonura, L. Spadaro, F. Frusteri, Journal of Catalysis 2007, 249, 185-194.
**[15]** J. Toyir, P. R. de La Piscina, J. L. G. Fierro, N. Homs, Applied Catalysis B: Environmental 2001, 29, 207-215.
**[16]** M. V. Twigg, M. S. Spencer, Applied Catalysis A: General 2001, 212, 161-174.

## Patentansprüche

1. Verfahren zur *in-situ* Umwandlung von chemisch fixiertem Kohlendioxid (CO₂) in organische Verbindungen, die 1 oder 2 Kohlenstoffatome und Wasserstoffatome enthalten, wobei einzelne Wasserstoffatome durch Hydroxyl-Gruppen oder durch Carbonyl-Gruppen substituiert sein können, wobei
a) CO₂ in ein organisches, CO₂-bindende Komponenten enthaltendes Waschreagenz, umfassend ein Alkoholamin und/oder Amin und/oder Arylamin und/oder beliebige Mischungen aus diesen, eingeleitet oder das organische, CO₂-bindende Komponenten enthaltende Waschreagenz mit CO₂ beaufschlagt wird, wobei das CO₂ an das organische, CO₂-bindende Komponenten enthaltende Waschreagenz chemisch fixiert und
b) direkt katalytisch oder elektrochemisch
in Gegenwart eines heterogenen Kupfer-basierenden Katalysators hydriert wird, wobei als Kathoden Kupfer-basierende oder übergangsmetallhaltige Elektrodenmaterialien eingesetzt werden, die Metalle der achten, neunten oder zehnten Gruppe des Periodensystems der Elemente aufweisen, und wobei im Anschluss die organischen Verbindungen, die 1 oder 2 Kohlenstoffatome und Wasserstoffatome enthalten, wobei einzelne Wasserstoffatome durch Hydroxyl-Gruppen oder durch Carbonyl-Gruppen substituiert sein können, von dem organischen, CO₂-bindende Komponenten enthaltenden Waschreagenz getrennt werden.

2. Verfahren nach Anspruch 1, **dadurch gekennzeichnet, dass** das organische, CO₂-bindende Komponenten enthaltende Waschreagenz substanziell wasserfrei ist.

3. Verfahren nach einem der Ansprüche 1 bis 2, **dadurch gekennzeichnet, dass** die Reaktionstemperatur bei der *in-situ* Umwandlung von chemisch fixiertem Kohlendioxid im Bereich zwischen 0 und maximal 200°C liegt.

4. Verfahren nach einem der Ansprüche 1 bis 3, **dadurch gekennzeichnet, dass** während der Umwandlung von chemisch fixiertem Kohlendioxid eine Abtrennung des als Produkt gebildeten Wassers erfolgt.

5. Verfahren nach einem der Ansprüche 1 bis 4, **dadurch gekennzeichnet, dass** bei der Umsetzung des chemisch fixierten CO₂ durch den Einsatz heterogener Metallkatalysatoren Wasserstoff als Reduktionsmittel mit einem Wasserstoffdruck zwischen 1 bar bis 200 bar in das organische, CO₂-bindende Komponenten enthaltende Waschreagenz eingeleitet wird.

6. Verfahren nach einem der Ansprüche 1 bis 5, **dadurch gekennzeichnet, dass** zur elektrochemischen Hydrierung mehrteilige Elektrolysezellen verwendet werden, bei denen die Elektrolysezellen durch eine protonenpermeable (H⁺) Membran unterteilt sind.

## Claims

1. Method for in-situ conversion of chemically fixed carbon dioxide (CO₂) into organic compounds which contain 1 or 2 carbon atoms and hydrogen atoms, wherein single hydrogen atoms can be substituted by hydroxyl groups or by carbonyl groups, wherein
a) CO₂ is introduced into an organic washing reagent that contains CO₂-binding components and comprises an alcohol amine and/or amine and/or aryl amine and/or any mixtures thereof, or CO₂ is supplied to the organic washing reagent that contains CO₂-binding components, wherein the CO₂ is chemically fixed to the organic washing reagent that contains CO₂-binding components, and
b) is directly catalytically or electrochemically hydrogenated in the presence of a heterogeneous, copper-based catalyst, wherein copper-based or transition-metal-containing electrode materials are used as cathodes, which materials comprise metals of the eighth, ninth or tenth group of the periodic table of elements, and wherein the organic compounds which contain 1 or 2 carbon atoms and hydrogen atoms, wherein single hydrogen atoms can be substituted by hydroxyl groups or by carbonyl groups, are subsequently separated from the organic washing reagent that contains CO₂-binding components.

2. Method according to claim 1, **characterised in that** the organic washing reagent that contains CO₂-binding components is substantially anhydrous.

3. Method according to either claim 1 or claim 2, **characterised in that** the reaction temperature for the in-situ conversion of chemically fixed carbon dioxide is in the range of between 0 and at most 200°C.

4. Method according to any of claims 1 to 3, **characterised in that**, during the conversion of chemically fixed carbon dioxide, the water formed as a product is separated off.

5. Method according to any of claims 1 to 4, **characterised in that**, when the chemically fixed CO₂ is transformed by using heterogeneous metal catalysts, hydrogen is introduced into the organic washing reagent that contains CO₂-binding components as a reducing agent having a hydrogen pressure of between 1 bar and 200 bar.

6. Method according to any of claims 1 to 5, **characterised in that** multi-part electrolytic cells are used for the electrochemical hydrogenation, in which multi-part electrolytic cells the electrolytic cells are divided by a proton-permeable (H⁺) membrane.

## Revendications

1. Procédé de conversion in situ de dioxyde de carbone (CO₂), fixé chimiquement, dans des composés organiques contenant 1 ou 2 atomes de carbone et des atomes d'hydrogène, des atomes d'hydrogène individuels pouvant être substitués par des groupes hydroxyle ou des groupes carbonyle,
a) le CO₂ étant introduit dans un réactif de lavage organique contenant des composants qui se lient au CO₂ et comprenant une alcoolamine et/ou une amine et/ou une arylamine et/ou des mélanges quelconques de celles-ci, ou le réactif de lavage organique, contenant des composants qui se lient au CO₂, étant chargé avec du CO₂, le CO₂ étant fixé chimiquement au réactif de lavage organique contenant des composants qui se lient au CO₂ et
b) étant hydrogéné directement par voie catalytique ou électrochimique en présence d'un catalyseur hétérogène à base de cuivre, des matériaux d'électrodes à base de cuivre ou de métaux de transition étant utilisés comme cathodes, lesquels matériaux comprenant des métaux du huitième, neuvième ou dixième groupe du tableau périodique des éléments, puis les composés organiques contenant 1 ou 2 atomes de carbone et des atomes d'hydrogène, des atomes d'hydrogène individuels pouvant être substitués par des groupes hydroxyle ou par des groupes carbonyle, étant séparés du réactif de lavage organique contenant des composants qui se lient au CO₂.

2. Procédé selon la revendication 1, **caractérisé en ce que** le réactif de lavage organique, contenant des composants qui se lient au CO₂, est sensiblement anhydre.

3. Procédé selon l'une des revendications 1 à 2, **caractérisé en ce que** la température de réaction lors de la conversion in situ du dioxyde de carbone fixé chimiquement est comprise entre 0 et 200 °C maximum.

4. Procédé selon l'une des revendications 1 à 3, **caractérisé en ce qu'**une séparation de l'eau formée comme produit est effectuée lors de la conversion du dioxyde de carbone fixé chimiquement.

5. Procédé selon l'une des revendications 1 à 4, **caractérisé en ce que** lors de la conversion du CO₂ fixé chimiquement par l'utilisation de catalyseurs métalliques hétérogènes, de l'hydrogène est introduit comme agent réducteur à une pression d'hydrogène comprise entre 1 bar et 200 bars dans le réactif de lavage organique contenant des composants qui se lient au CO₂.

6. Procédé selon l'une des revendications 1 à 5, **caractérisé en ce que** des cellules d'électrolyse en plusieurs parties, lesquelles cellules d'électrolyse sont subdivisées par une membrane perméable aux protons (H⁺), sont utilisées pour l'hydrogénation électrochimique.
